(19) Europäisches Patentamt
European Patent Office
Office européen des brevets

(11) EP 0 953 726 B1

(12) EUROPEAN PATENT SPECIFICATION

(45) Date of publication and mention of the grant of the patent:
**08.06.2005 Bulletin 2005/23**

(51) Int Cl.$^7$: **E21B 49/10**, G01H 5/00, G01N 29/02, G01N 33/28

(21) Application number: **98302530.5**

(22) Date of filing: **01.04.1998**

# (54) Apparatus and method for wellbore testing of formation fluids using acoustic signals

Vorrichtung und Verfahren zur Untersuchung von Formationsflüssigkeiten in einem Bohrloch mittels akustischer Signale

Dispositif et procédé pour l'essai de fluides de formation dans un puit au moyen de signaux acoustiques

(84) Designated Contracting States:
**DE FR GB**

(43) Date of publication of application:
**03.11.1999 Bulletin 1999/44**

(73) Proprietor: **Halliburton Energy Services, Inc.**
**Duncan, Oklahoma 73536 (US)**

(72) Inventors:
- **Birchak, James R.**
  **Spring, Texas 77388 (US)**
- **Proett, Mark A.**
  **Houston, Texas 77459 (US)**

(74) Representative: **Wain, Christopher Paul et al**
**A.A. Thornton & Co.**
**235 High Holborn**
**London WC1V 7LE (GB)**

(56) References cited:
**US-A- 4 571 693**
**US-A- 5 473 939**
**US-A- 5 635 631**
**US-A- 5 335 542**
**US-A- 5 583 280**
**US-E- R E34 975**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

This invention relates generally to formation fluid testing in a wellbore and, more particularly, to a closed-loop system for in situ determining the type and condition of formation fluids in a wellbore and optionally for collecting downhole formation fluid samples under the original formation conditions.

In the oil and gas industry, wireline formation testing tools have been used for monitoring formation pressures, obtaining formation fluid samples and for predicting reservoir performance. Such formation testing tools typically contain an elongated body having an elastomeric packer that is sealingly urged against the zone of interest in the wellbore to collect formation fluid samples in storage chambers placed in the tool.

Various types of drilling fluids are used to facilitate the drilling process and to maintain a desired hydrostatic pressure in the wellbore. These drilling fluids penetrate into or invade the formations for varying radial depths (referred to generally as the invaded zones) depending upon the types of the formation and drilling fluid used. Any initial fluid collected by the formation testing tools must first be analyzed to determine when the formation fluid being withdrawn is substantially free of mud filtrates and, thus, to collect only the uncontaminated fluid. Additionally, it is desirable to collect the formation fluids for further analysis in the same condition they exist in the formation. This typically requires that the fluid drawdown pressure be maintained above the bubble point of the fluids. The formation testing tools have utilized various sensors and in situ techniques to determine when the formation fluids being withdrawn are substantially free of mud filtrates and to maintain the drawdown pressure above the bubble point so as to collect clean fluids under the original formation conditions.

Resistivity measurements, downhole pressure and temperature measurements, and optical analysis of the formation fluids have been used to identify the type of formation fluid, i.e., to differentiate between oil, water and gas present in the formation fluid and to determine the bubble point pressure of the fluids. The information obtained from one or more pressure sensors and temperature sensors, resistivity measurements and optical analysis is utilized to control the drawdown rate so as to maintain the drawdown pressure above the bubble point and to determine when to collect the fluid samples downhole.

US Re. 34,975 describes an apparatus for ultra-sonic measurement of borehole characteristics while a well is being drilled.

Prior art tools have utilized resistivity measuring devices and temperature sensors as part of a probe assembly to monitor the formation fluid characteristics. Optical fluid analyzers utilizing near-infrared spectroscopy absorption and reflection to differentiate between oil, water and gas have also been used. The fluid from the formation is discharged into the wellbore until the fluid flowing through the flowline is determined to be substantially free from contaminants. The fluid from the formation is discharged into the wellbore until the fluid flowing through the flowline is determined into the wellbore until the fluid flowing through the flowline is determined to be substantially free from contaminants. The fluid drawrate from the formation is controlled to maintain the drawdown pressure above the bubble point.

The interpretation of the flowline resistivity is difficult and often inaccurate. Interpretation of the resistivity must take fluid dynamics into consideration. The resistivity measured is that of the continuous phase of the fluid in the flowline. A water/hydrocarbon mixture with the water as the continuous phase has a low resistivity that increases due to tortuosity as the percentage of hydrocarbon increases. A water-hydrocarbon mixture with hydrocarbon as the continuous phase can have a high resistivity even if the water volume is large. Flow of alternating slugs of hydrocarbon and water produces noisy resistivity recording. This effect is more evident when gas is present. The optical analyzer provides more accurate results, but is quite expensive and requires the use of sophisticated electronics downhole, which must operate at very high temperatures. Additionally, optical devices tend to become clouded with a thin film which reduces the measurement accuracy.

Thus, a need exists to provide a formation fluid retrieval and collection system that is relatively simple, less expensive than the current state-of-the-art systems and relatively accurate in differentiating between the various types of fluid conditions to ensure that substantially uncontaminated formation fluid samples are collected and that the drawdown pressure is maintained above the bubble point of the formation fluid during the fluid collection process.

The present invention addresses the above-noted deficiencies and provides a relatively simple closed loop system for collecting one or more formation fluid samples under original formation conditions using a novel acoustic cell technique.

In one aspect, the invention provides apparatus for testing formation fluid from a wellbore formation, comprising:

(a) a probe adapted for placement against the formation for withdrawing the formation fluid into a flowline used for retrieving said formation fluid;
(b) a pump for controlling the flow rate of the formation fluid into the flowline; and
(c) an acoustic device for providing signals for determining characteristics of the formation fluid in the flowline, said acoustic device having a transducer for transmitting acoustic signals through the fluid in the flowline and a receiver

for detecting acoustic signals passing through the fluid in the flowline.

In another aspect, the invention provides a system for analysing and collecting formation fluid from an apparatus of the invention, comprising the apparatus of the invention and a surface control unit coupled to the apparatus, said surface control unit having a computer for receiving the processed acoustic signals from the apparatus and determining the fluid density and fluid compressibility therefrom, said control unit transmitting command signals to apparatus for controlling the flow rate into the flowline as a function of the fluid density and compressibility measurements so as to maintain the pressure in the flowline above the bubble point Pressure of any gas contained in the formation fluid; and a two-way telemetry system for transmitting data between the downhole formation testing tool and the surface control unit.

The invention further provides a method of retrieving a formation fluid from a zone of interest in a wellbore, said method comprising the steps of:

(a) withdrawing formation fluid from the zone of interest into a flowline;
(b) determining the speed of sound in the fluid, acoustic impedance and acoustic absorption coefficient of the fluid flowing through the flowline;
(c) determining the density and compressibility of the fluid from the speed of sound in the fluidacoustic impedance and the acoustic absorption coefficient of the fluid flowing through the flowline; and
(d) controlling the flow of the fluid into the flowline based on the fluid density and the compressibility of the fluid flowing through the flowline so as to ensure that the pressure in the flowline remains above the bubble point of the fluid.

This invention can utilise a closed-loop system for withdrawing a formation fluid from a zone of interest in a wellbore, in situ determination of the type (single phase or two phase) of the formation fluid being withdrawn, determining the bubble point pressure of the fluid, and for selectively collecting fluid samples above the bubble point pressure of the fluid that are substantially free from any mud filtrates. The closed loop system preferably contains an elongated member which has at least one probe that is adapted to be sealingly placed against the wellbore formation. A pump coupled to the probe remotely controls the flow of a fluid from the wellbore formation into a flowline in the elongated member. A pressure sensor preferably provides downhole hydrostatic pressure and an acoustic density cell preferably provides the speed of sound in, acoustic impedance of, and acoustic absorption coefficient of the fluid in the flowline. In one arrangement, the system determines the density and compressibility of the formation fluid in the flowline from the speed of sound and the acoustic impedance of the fluid and, in response thereto, controls the fluid flow into the flowline for example to selectively collect the formation fluid samples that are substantially free from any mud filtrates. The acoustic absorption coefficient is very sensitive to bubbles and aids in maintaining the fluid pressure above the bubble point pressure of the fluid. The absorption coefficient is also sensitive to the fluid viscosity which aids in distinguishing among hydrocarbons.

The apparatus of the invention may be used in a method for retrieving and collecting formation fluids from a zone of interest in a wellboreat the original formation conditions. This method contains the steps of: (a) sealingly placing the probe against the zone of interest in the wellbore for receiving the formation fluid; (b) controllably allowing the fluid to pass from the probe into a flowline; (c) determining the speed of sound in the fluid, the acoustic impedance and acoustic absorption coefficient of the fluid in the flowline; (d) determining the density and compressibility of the fluid from the speed of sound in the, acoustic impedance and acoustic absorption coefficient of the fluid in the flowline; (e) controlling the flow of the fluid into the flowline based on the fluid density and compressibility so as to maintain the fluid pressure in the flowline above the bubble point pressure of the fluid; and (f) collecting a sample of the fluid from the flowline into a downhole storage chamber above the bubble point pressure of the fluid.

For detailed understanding of the present invention, reference should be made to the following detailed description of various preferred embodiments, taken in conjunction with the accompanying drawings, in which like elements have been given like numerals, and wherein:

**FIG. 1A** shows a schematic elevational view of a system having one embodiment of a sectional formation evaluation tool conveyed in a wellbore for testing and retrieving formation fluids according to the present invention.
**FIG. 1B** shows a schematic elevational view of the system of **FIG. 1A** utilizing an alternative sectional arrangement for the formation evaluation tool.
**FIG. 1C** shows a schematic elevational view of the system of **FIG. 1A** utilizing a further alternative sectional arrangement for the formation evaluation tool.
**FIG. 2** shows an elevational cross-section of an embodiment of the sonic density cell suitable for use in the formation evaluation tool according to the present invention.
**FIG. 3** shows an elevational cross-section of another embodiment of the sonic density cell for use in the formation

evaluation tool.

**FIG. 4A** shows typical waveforms of input electrical signal to and generated acoustic pressure wave by the transmitter **108** of the density cells, and waveform of the acoustic echo signal received by the receiver of the density cells shown in **FIG 2**.

**FIG. 4B** shows waveforms of received acoustic pressure wave and the resulting electrical voltage signal produced by transducer **116** of **FIG 2**.

**FIG. 5** shows a typical plot of the acoustic absorption coefficient ($\alpha$) as a function of the percentage of gas phase content in a fluid.

**FIG. 6** shows a block diagram of the circuit for transmitting, receiving and recording signals to and from the sonic density cell and other modules of the tool.

Referring to the drawings, **FIG. 1A** shows an embodiment of a wireline formation evaluation and testing system **10** having a downhole formation evaluation and testing tool (apparatus) **24** conveyed in a wellbore **21** by a wireline **23'** for testing and retrieving formation fluids from desired formations within the wellbore **21** according to the normal operation of the system **10**. The tool **24** contains a number of functional modules which are serially coupled to form the tool **24** that suits the needs in a particular test situation. In the embodiment of **FIG. 1A**, the tool **24** includes a sequential arrangement of an electro-hydraulic system **25**, a packer/probe module **26**, an acoustic density cell module **15**, a flushing pump module **12**, a sampling module **16**, and a test equalization valve **20**. The tool **24** is conveyed in the wellbore by the wireline (cable) **23'** which contains a plurality of conductors for providing power to the various components in the tool **24** and for providing two-way electrical and data communication between the tool **24** and a control unit **50**, which is usually placed uphole in a suitable truck **60** for land operations and in a cabin (not shown) for offshore operations. The wireline **23'** is conveyed by a drawworks **62** via a system of pulleys **22a** and **22b.**

The control unit **50** contains a computer and associated memory for storing therein desired programs and models. The control system **50** controls the operation of the tool **24** and processes online data received from the tool **24**during operation of the tool **24**. The control unit **50** is coupled to a variety of peripherals, such as a recorder **52** for recording data and a display/monitor **54** for displaying desired information during operation. The use of the control unit **50**, display/monitor **54** and recorder **52** is known in the art of well logging and is, thus, not explained in greater detail herein.

**FIG. 1B** shows a schematic view of an alternative arrangement of the various sections of the tool **24**. In this embodiment, the packer/probe section **26** is placed near the bottom of the tool string for rathole type testing. The sequential arrangement of the sections contains the test equalization valve **20**, sampling section **16**, flushing pump section **12**, sonic density cell section **15**, electro-hydraulic system **25**, and formation tester packer/probe section **26**.

**FIG. 1C** shows a schematic view of the system of **FIG. 1A** having yet another arrangement of the various tool sections. The sequential order of the sections in the embodiment of **FIG. 1C** is the same as that of the embodiment shown in **FIG. 1A** except that the packer/probe section **26** contains multiple packer/probe units and utilizes inflatable packers as the backup pads.

Referring back to **FIG. 1A**, the formation tester packer/probe section **26**, contains one or more inflatable packers/probes **29**, each such packer/probe being adapted to be sealingly urged against the wellbore wall **21a**. One or more backup pads **27** are urged against the wellbore wall **21a** opposite the packer/probe **29**. The electro-hydraulic section **25** is utilized to inflate the pads of the packer/probe **29** to seal it against the interior of the wellbore **21**. The electro-hydraulic section **25** also deploys backup pads **27** to cause the packer/probe **29** to urge against the wellbore wall **21a**. Any other suitable means may also be used for deploying packer/probe section **26** for the purposes of this invention. Each packer/probe section **26** contains a probe (not shown) radially extending away from the tool body and is adapted to penetrate into the formation **22** when the packer/probe **29** is urged against the wellbore interior **21a**. The packer/probe section **26** also contains a pressure gauge **28** to monitor pressure changes during fluid sample collection process, an isolation valve **11** for use during flushing operations, and a pretest piston **30** for drawing samples during pretesting.

Still referring to **FIG. 1A**, the acoustic density cell **15** is adapted to receive fluid flow through the probe of packer/probe **29**, which is more fully explained later in reference to **FIG. 2**. A flushing pump section **12** containing a flushing pump **13** is adapted to receive the fluid passing through the acoustic cell **15** via a flowline **14** where the fluid is directed in to the sampling section **16**. The sampling section **16** contains a sample chamber valve **17**, which is opened to direct the formation fluid to one or more sample chambers **18** for collecting formation fluid therein. When the sample chamber valve **17** is closed, the fluid is discharged into formation borehole **21**. The sample chamber valve **17** may be controlled downhole or from the surface by the control unit **50**. An equalization valve **20** is provided to maintain a desired pressure in the flowline **14**. The equalization valve is closed during sample collection and closed during the flushing operation. A piston **19** is used to control the fluid pressure during sample collection to assure that the sample fluid does not separate out in to two phases,i.e., gas and liquid. Different sections of the tool **24** are suitably connected to each other by means of section connectors, generally designated by numeral **23**.

**FIG. 2** shows an elevational cross-section of one embodiment of an acoustic density cell **100** according to the present invention. The acoustic density cell **100** contains a delay line **104** of thickness $\mathbf{X_{dl}}$ having opposing surfaces **103** and **105**. The surface **103** is in close acoustic contact with a fluid flowline **102** through which the formation fluid flows during the testing and collection of the formation fluid. The delay line **104** material may be a machineable glass or any other suitable material that would sufficiently delay the acoustic signal passing therethrough, preferably by a time that exceeds the transducer ringdown time, and have an acoustic impedance between the impedance of the piezoelectric material used in the transducer and the formation fluid. Such a delay line enables measuring the acoustic impedance of the fluid. An acoustic transducer **108** (transducer 1) of thickness **L2** having a opposing surfaces **107** and **109** is placed against the delay line **104**. The transducer **108** may be used as an acoustic signal transmitter or a receiver. The transducer **108** preferably is made of a suitable piezoelectric material but is not limited to such a material. The surface **105** of the delay line **104** and the surface **107** of the transducer **108** are preferably bonded together. A backing **112** of thickness $\mathbf{L_1}$ is placed against transducer **108** so that the surface **111** of the backing is in contact with the transducer **108**. The backing **112** is preferably made of a mixture of tungsten and a rubber material but is not limited to such a composition. The backing material **112** preferably has a high acoustic absorption coefficient and an acoustic impedance that will minimize the acoustic reflection from the surface **111**. The thickness $\mathbf{L_1}$ is made sufficiently large so as to minimize reflections from the surface **113.** The surface **109** of the transducer **108** and the surface **111** of the backing **112** are preferably bonded together.

A second acoustic transducer **116** (transducer 2) of thickness $\mathbf{L_2}$ having opposing surfaces **115** and **117** is placed against the flowline **102** across from the delay line **104.** The transducer **116** also is preferably made of piezoelectric material but may be made of any other suitable material. The surface **115** of the transducer **116** is in close acoustic contact with the fluid flowline **102**. A backing **120** of thickness $\mathbf{L_3}$ having opposing surfaces **119** and **121** is placed against the transducer. The surface **117** of the transducer **116** and the surface **119** of the backing **120** are preferably bonded together. In the preferred embodiment, the delay line **104**, first transducer **108**, backing **112**, second transducer **116**, and backing **120** preferably have the same depth **D**.

**FIG. 3** shows an elevational cross-section of an alternative embodiment of the sonic density cell **100** according to the present invention. In this embodiment, the elements shown on the left of the fluid flowline **102** in **FIG. 3** are identical to the elements shown in **FIG. 2**. The elements to the right of the fluid flowline **102** in **FIG. 3** are a mirror image of the elements to the left of the fluid flowline **102**. In the embodiment shown in **FIG. 3**, a delay line **124** of thickness $\mathbf{X_{dl}}$ has a surface **123** in close acoustic contact with the fluid flowline **102** opposite the delay line **102**. A backing **120** of thickness **L1** having surfaces **119** and **121** is placed against the transducer **116**. The surface **115** of the transducer **116** is preferably bonded to the surface **125** of the delay line **124**. The surface **117** of the transducer **116** and the surface **119** of the backing **120** are preferably bonded together. The advantages of this embodiment are that the transducers **108** and **116** are isolated from the fluid, the two sides of the sonic density cell are identical in design and the acoustic impedance of the fluid may be averaged for surfaces **103** and **123**. The overall size of the sonic density cell **100**, however, is somewhat increased.

The dimensions of the elements in the preferred embodiment of the sonic density cell **100** are (in inches): $\mathbf{L_1}$ = **2.0,** $\mathbf{L_2}$ = **0.1**, $\mathbf{X_{dl}}$ = **0.6,** $\mathbf{X_n}$ = **0.25, and D = 1.0.** The signal frequency applied to the transmitters may be varied from **0.3** to **10** mega hertz (MHz) depending on the materials used and the dimensions of the elements in constructing the sonic density cell **100.** For a single phase flow in the flowline **102** the frequency of the signals transmitted through the fluid from one transducer to the other may be varied to obtain the viscosity of the fluid. For two phase flow, viscosity and also scattering from the second phase in the continuous phase affect the frequency of signals passing through the fluid. The dimensions and signal frequencies indicated herein can be easily varied to suit different test scenarios and they are not intended as any limitations on the embodiment of the sonic density cell **100**.

**FIG. 4A** shows typical waveforms of input electrical signal, generated acoustic pressure wave, and received acoustic echo signal for the transmitter/receiver transducer **108** of **FIG. 2**. Now referring to **FIGS. 2**, **4A**, and **4B**, when an electrical signal of amplitude $\mathbf{V_0}$ is applied to the transducer **108**, an acoustic pressure wave shown in **FIG.4A** is produced after a short time delay ($\mathbf{t_{01}}$). This acoustic pressure wave travels in all adjacent media. Whenever an acoustic wave encounters a new medium, a fraction of the wave amplitude is reflected and a fraction of the wave amplitude is transmitted through the new medium. The reflected wave from the interface of the delay line **104** and the test fluid flowing through the fluid flowline **102** provides a measure of the acoustic impedance of he fluid. The resultant echo signal of amplitude $\mathbf{V_{pp1}}$ is received by the transducer **108** at time $\mathbf{t_1}$. The time difference $\mathbf{(t_1 - t_{01})}$ is related to the speed of sound in the delay line **104**. The signal amplitude $\mathbf{V_{pp1}}$ of the echo is also related to the acoustic impedances of the material of the delay line **104** $\mathbf{(Z_{dl})}$ and that of the fluid interface $\mathbf{(Z_n)}$. After the acoustic wave transmitted into the fluid travels through the fluid, the second transducer **116** detects its arrival at time $\mathbf{t_2}$ with an amplitude $\mathbf{V_{pp2}}$.

A short time delay $\mathbf{t_{02}}$ occurs between the arrival of the acoustic wave at the transducer **116** and the electronic detection of the signal. The time $\mathbf{t_2}$ is related to the speed of sound through the delay line **104** and the speed of sound through the fluid path in the fluid flowline **102**. The amplitude $\mathbf{V_{pp2}}$ of the signal received by the transducer **116** is related to the acoustic signal transmission and attenuation through the delay line **104** and through the fluid in the fluid flowline

**102**.

Physical properties of the delay line **104** material may be determined in the laboratory by known calibration procedures. Utilizing the acoustic density cell **100** measurements and the parameters of the properties of the delay line **104**, the density and compressibility of the test fluid may be determined using equations **1** through **14** given below. Also, acoustic attenuation coefficient ($\alpha$) of the test fluid may be determined using the acoustic density cell **100** measurements and equations **15** through **18** given below.

**Fluid Density and Compressibility Calculations:**

Acoustic impedance for fluid and delay line:

$$Z_{fl} = c_{fl}\rho_{fl} \qquad 1.)$$

$$Z_{dl} = c_{dl}\rho_{dl} \qquad 2.)$$

Acoustic sound speed and wave length for fluid and delay line material:

$$c_{fl} = \frac{x_{fl}}{t_{fl}} \qquad 3.)$$

$$c_{dl} = \frac{x_{dl}}{t_{dl}} \qquad 4.)$$

$$\lambda_{fl} = \frac{c_{fl}}{f_{fl}} \qquad 5.)$$

$$\lambda_{dl} = \frac{c_{dl}}{f_{dl}} \qquad 6.)$$

Relationship of the echo time measured by transducer 1 to the delay time:

$$t_1 = 2\cdot(t_{01} + t_{dl}) \qquad 7.)$$

Solving for delay line time:

$$t_{dl} = \frac{t_1}{2} - t_{01} \qquad 8.)$$

Relationship of the received signal in transducer 2 to the fluid time and delay time:

$$t_2 = t_{01} + t_{dl} + t_{02} + t_{fl} = \frac{t_1}{2} + t_{02} + t_{fl} \qquad 9.)$$

Solving for the fluid time:

$$t_{fl} = t_2 - \frac{t_1}{2} - t_{02} \qquad 10.)$$

The reflectance measured by transducer 1:

$$R_1 = \frac{V_{pp1}}{K_1 V_O} = \left(\frac{Z_{fl} - Z_{dl}}{Z_{fl} + Z_{dl}}\right) \quad 11.)$$

Solving for the fluid acoustic impedance:

$$Z_{fl} = Z_{dl}\left(\frac{1 + \frac{V_{pp1}}{K_1 V_O}}{1 - \frac{V_{pp1}}{K_1 V_O}}\right) \quad 12.)$$

Solving for the fluid density:

$$\rho_{fl} = \frac{Z_{fl}}{c_{fl}} = \frac{Z_{dl}}{c_{fl}}\left(\frac{1 + \frac{V_{pp1}}{K_1 V_O}}{1 - \frac{V_{pp1}}{K_1 V_O}}\right) = \rho_{dl}\frac{x_{dl}}{x_{fl}}\left(\frac{t_2 - \frac{t_1}{2} - t_{02}}{\frac{t_1}{2} - t_{01}}\right)\left(\frac{1 + \frac{V_{pp1}}{K_1 V_O}}{1 - \frac{V_{pp1}}{K_1 V_O}}\right) \quad 13.)$$

The f uid compressibility can now be determined from the density and sound speed

$$\gamma_{fl} = \frac{1}{\rho_{fl} c_{fl}^2} = \frac{1}{\rho_{fl}}\left(\frac{t_2 - \frac{t_1}{2} - t_{02}}{x_{fl}}\right)^2 \quad 14.)$$

**Fluid Attenuation and Calculations:**

Delay line to fluid acoustic transmission:

$$T_{dl\text{-}fl} = \frac{2 \cdot Z_{fl}}{Z_{dl} + Z_{fl}} \quad 15.)$$

Fluid to transducer 2 acoustic transmission:

$$T_{fl\text{-}2} = \frac{2 \cdot Z_2}{Z_{fl} + Z_2} \quad 16.)$$

Relationship of the measured peak to peak voltage received in transducer 2:

$$V_{pp2} = K_1 V_0 T_{dl\text{-}fl} e^{(-\alpha x_{fl})} \cdot K_2 T_{fl\text{-}2} \quad 17.)$$

Solving for the attenuation coefficient:

$$\alpha = -\left(\frac{1}{x_{fl}}\right)\ln\left(K_1 \cdot T_{dl-fl} \cdot K_2 \cdot T_{fl-2} \cdot \left(\frac{V_0}{V_{pp2}}\right)\right) \quad \text{.............................................. 18.)}$$

Nomenclature used herein is as follows:

Variables:

| | |
|---|---|
| *V* - | voltage |
| *c* - | speed of sound |
| $\gamma$ - | compressibility |
| $\lambda$ - | wave length |
| $\rho$ - | density |
| *f* - | frequency |
| *x* - | distance |
| Z - | acoustic impedance |
| R - | acoustic reflectance |
| T - | acoustic transmission |

Subscripts:

| | |
|---|---|
| 1 - | transducer 1, transmitter/receiver |
| 2 - | transducer 2, receiver |
| pp - | peak to peak voltage |
| fl - | fluid |
| dl - | delay line |

Calibration and known values:

| | |
|---|---|
| $c_{dl}$ - | sound speed of delay line material |
| $\rho_{dl}$ - | density of delay line material |
| $t_{01}$ - | offset time for transducer 1 electronic delay |
| $t_{02}$ - | offset time for transducer 22 electronic delay |
| $x_{dl}$ - | thickness of delay line |
| $x_{fl}$ - | thickness of fluid passage |
| $V_0$ - | calibration voltage |
| $K_1$ - | calibration constant for transducer 1 |
| $K_2$ - | calibration constant for transducer 2 |

Measured values:

| | |
|---|---|
| $t_1$ - | time for echo signal for transducer 1 |
| $t_2$ - | time for received signal for transducer 2 |
| $V_{pp1}$ - | peak to peak voltage for echo signal for transducer 1 |
| $V_{pp2}$ - | peak to peak voltage for received signal for transducer 2 |

The nomenclature used in the above equations is as follows The acoustic attenuation coefficient ($\alpha$) of a two phase fluid, e.g. liquid and gas, depends on the percentages of the two constituents. Acoustic attenuation coefficient of fluids containing known percentages of the two constituents can be determined in the laboratory. A graph plot of the laboratory measured acoustic attenuation coefficient as a function of the percentage of gas component in the fluid can be obtained. This acoustic attenuation depends on the viscosity, frequency and the size of the second phase in the

continuous phase.

**FIG. 5** shows a typical plot of the acoustic attenuation coefficient ($\alpha$) as a function of the percentage of gas phase content in a fluid when the other parameters remain constant. The fraction of the gas in the fluid $\beta$ can be determined numerically by making a polynomial curve fit of the laboratory measured data to the plot in **FIG. 5**. This polynomial fit is then compensated for the effects of viscosity and the second phase distribution. This compensation is preferably achieved by regression methods on known two-phase fluids. A typical formation is developed according to equation **(19)** given below. Thus, using measurements from the sonic density cell **100**, the acoustic absorption coefficient of the test fluid is calculated according to equation **(18)** described earlier, and then the fraction of the gas phase is determined according to equation **(19)**.

$$\beta = A_0 + B_0\alpha + C_0\alpha^2 + D_0\alpha^3 + E\,f_0 + F\,f_0^2 + G\,f_0^3 + H\,P_0 \qquad (19)$$

The above equation requires broadband acoustic transmitters and spectral analysis of the received signals to perform frequency **(f)** and pressure **(p)** compensation. The spectral dependance of $\alpha$ provides the spectral dependence of $\beta$. The empirical measurements on known two-phase fluids can be made in the laboratory to interpret the fraction of the gas phase from the frequency dependence of equation (19). The frequency dependancies of viscosity and two phase scattering differ. Equation **19** sibtracts viscosity effects to improve the accuracy of calculating $\beta$.

To operate the system of the present invention, the tool **24** is lowered into the wellbore **21** by means of the wireline **23'** to a desired depth. The packer/probe **29** is urged against the wall of the wellbore **21** at the zone of interest in the formation **22**. The electro hydraulic system **25** deploys the probe of the packer/probe **29** and backup pads **27** to create a hydraulic seal with the elastomeric packer/probe **29** against the formation **22**. Normally the tool equalization valve **20**, the sample chamber valve **17** and the isolation valve **11** remain closed during the packer/probe **29** deployment. The pretest piston **30** is used to draw a small sample of fluid into the flowline **14** of the tool **24** while the flowline **14** is monitored using a high accuracy quartz pressure gauge **8**. As the fluid sample is drawn into the flowline **14** of the tool **24** the pressure decreases due to the resistance of the formation **22** to fluid flow. When the pretest stops, the pressure in the flowline **14** increases until it equalizes with the pressure in the formation **22**. This is due to the formation **22** gradually releasing the fluids into the probe of the packer/probe **29**. The formation pressure is typically lower than the borehole **21** or hydrostatic pressure and this difference is used as a means for verifying that the packer/probe **29** is sealed against the wall of the borehole **21**. The fluids in the pore space of the formation **22** near the probe of the packer/probe **29** are typically invaded with the mud filtrate fluids.

To obtain a sample that is representative of the in situ formation fluids the area near the probe needs to be flushed or pumped until formation fluids are flowing into the tool **24**. To flush the area near the probe of the packer/probe **29**, the isolation valve **11** and the equalization valve **20** are opened, and the flushing pump **13** is started. The flushing pump **13** is preferably a double acting piston pump so that the fluid flow will be constant and as uniform as possible. However, this invention is not limited to a particular type of flushing pump and any suitable means may be used to serve the stated purpose. The flushing pump **13** draws fluid into the probe of the packer/probe **29** and expels fluid at hydrostatic pressure out of the equalization valve **20.**

The pumping rate of the flushing pump **13** is regulated such that the pressure in the flowline **14** near the probe is maintained above the bubble point of the fluid sample. While the flushing pump is running, the sonic density cell **15** is used to measure the fluid properties. The sonic density cell **15** can monitor the density of the fluid, sound speed in the fluid, bulk modulus of the fluid, and presence of gas bubbles in the fluid. By monitoring the formation of gas bubbles in the fluid, the flow in the flowline **14** can be constantly adjusted so that a single phase fluid is maintained by regulating speed of the flushing pump **13**.

The aforementioned fluid properties are of the type of fluid in the flowline **14** and knowledge of the fluid properties can be used to monitor the fluid flow while the formation fluid is being pumped for sample collection. Thus when it is determined that the formation fluid flowing through the flowline **14** is representative of the in situ conditions, the sample chamber valve **17** is opened and the equalization valve **20** is closed. The flushing pump **13** continues to pump the formation fluid through the flowline **14** and the fluid is directed into the sample chamber **18** until it is filled. The flushing pump **13** may continue to pressure the collected sample in the sample chamber **18** to a desired pressure level to assure in situ pressure level, and then all of the valves, the isolation valve **11**, the sample chamber valve **17**, and the equalization valve **20** are closed.

The bubble point of the fluid in the flowline **14** is measured by using the sonic density cell **15** in conjunction with the flushing pump **13**. The flushing pump **13** is used to lower the pressure in the flowline **14** between the isolation valve **11** and the flushing pump **13**. The sonic density cell **15** determines the density change of the fluid and bubble formation in the flowline **14**. A pressure gauge (not shown) is also used to determine the bubble point of the fluid.

The configuration of various sections in the tool **24** may be changed to suit the test conditions but it is desirable

for the sonic density cell **15** to be placed between the flushing pump **13** and the packer/probe section **29** to enable monitoring of the fluid properties on the low pressure side of the flushing pump **13**. As discussed earlier, **FIG. 1B** shows an alternative embodiment of the tool **24** wherein the packer/probe section **29** is near the bottom of the string of the sections in tool **24** for rathole type testing. **FIG. 1C** shows yet another embodiment of the tool **24** with an inflatable packer section of the packer/probe **29** rather than a single packer/probe unit. Additionally, multiple packer/probe sections can be used instead of a single packer/probe section **29** but the basic operation of the tool **24** remains the same.

**FIG. 6** shows a functional block diagram for recording signals from the sonic density cell **15** and other tool sections. Referring now to **FIGS. 1A**, **2, 4A, 4B** and **6**, the truck logging processor **240** contains a control unit **50** for controlling the operation of the tool **24**, a display monitor **52** for displaying the operator commands and the real time results from the tool **24**, and a recorder **54** that records desired signals to and from the tool **24**. A downhole microprocessor **200** (preprogrammed or operator controlled) instructs the signal input generator **210** to apply electrical signal shown in **FIG 4A** to transducer 1 **108**. The echo signal received by transducer **108** after time $\mathbf{t_1}$ and electrical signal generated by the transducer **116** after time $\mathbf{t_2}$ are communicated to the downhole microprocessor **200**. The downhole microprocessor **200** computes the fluid density, the fluid compressibility, percentage of gas content in the test fluid and communicates the signals to signal processor **230**. Information received by the signal processor is communicated to the truck logging processor **240** where it processed, recorded and monitored by the testing personnel.

As noted earlier, the initial fluid drawn from the formation **22** typically contains mud filtrates which have invaded into the formation **22**. It is, therefore, important that the formation fluids collected downhole be uncontaminated (clean fluid) and in the same physical conditions in which such fluids are present in the formation. For example, the gas and oil contents of the fluid should be maintained in the manner present in the formation during the collection process. This requires determining when the fluid flowing through the flowline is substantially free of mud filtrates and collecting the fluid above the bubble point pressure of the fluid. As noted earlier, the prior art tools utilize resistivity measurements and optical means to continuously determine the downhole conditions of the fluid during the collection process and to control the flow rate to maintain the fluid pressure above the bubble point. Such apparatus and method are complex and are usually are very expensive, susceptible to contamination and very reliable. When a desired amount of the fluid has been collected in the fluid chamber **18**, a new command signal may be sent downhole to select other chambers or to stop the operation of the pump **17** or to reverse the cycle and start another cycle, if necessary.

To determine the bubble point pressure, the formation fluid flow rate into the flowline **14** is slowly increased by controlling the flush-pump **13** while continuously monitoring the fluid density and the fluid compressibility. As the fluid rate is increased, the gas in fluid, if present, will expand into a gaseous state from its normal liquid state which it is present in the formation, which will be observed as a sudden decrease in the density. The pressure at which the density drops is the bubble point pressure of the fluid. To ensure the accuracy of the results, the flow rate is decreased until the density suddenly rises to the initial value of the clean fluid and the corresponding fluid pressure. The procedure may be repeated if necessary to accurately determine the bubble point pressure.

## Claims

1. Apparatus for testing formation fluid from a wellbore junction, comprising:

   (a) a probe (29) adapted for placement against the formation (22) for withdrawing the formation fluid into a flowline (14; 102) used for retrieving said formation fluid;
   (b) a pump (13) for controlling the flow rate of the formation fluid into the flowline (14; 102); and
   (c) an acoustic device (15; 100) for providing signals for determining characteristics of the formation fluid in the flowline (14; 102), said acoustic device (15; 100) having a transducer (108) for transmitting acoustic signals through the fluid in the flowline (14; 102) and a receiver (116) for detecting acoustic signals passing through the fluid in the flowline (14; 102).

2. Apparatus according to claim 1, wherein the acoustic device (15; 100) further has a first delay line (104) placed between the acoustic transmitter (108) and the flowline (14; 102) so as to cause the acoustic signals to pass through the delay line (104) before reaching the flowline (14; 102).

3. Apparatus according to claim 2, wherein the acoustic device (100) further has a second delay line (124) placed between the acoustic receiver (116) and the flowline (14; 102) so as to cause the acoustic signals form the acoustic transmitter (108) to pass through the delay line (124) before reaching the receiver (116).

4. Apparatus according to claim 3, wherein the delays in the first (104) and second (124) delay lines are substantially identical in composition and size.

**5.** Apparatus according to any of claims 1 to 4, further having a control circuit (200) for determining the characteristics of the fluid in the flowline (14; 102), said control circuit (200) preferably controlling the flow of the fluid into the flowline (14; 102) as a function of the determined characteristics of the fluid, said fluid characteristics preferably being density of the fluid in the flowline (14; 102) or compressibility of the fluid in the flowline (14; 102).

**6.** Apparatus according to claim 5, wherein the control circuit (200) controls the flow of the formation fluid so as to maintain the pressure in the flowline (14; 102) above the bubble point pressure of any gas in the formation fluid in the flowline (14; 102).

**7.** Apparatus according to claim 6, wherein the control circuit (200) is operatively coupled to the pump (13) and controls the pump to control the formation fluid flow rate into the flowline (14; 102) as a function of the fluid density and compressibility so as to maintain the flowline pressure above the bubble point pressure of any gas contained in the formation fluid in the flowline (14; 102).

**8.** Apparatus according to any of claims 1 to 7, further having a chamber (18) for receiving formation fluid from the flowline (14; 102), said apparatus further preferably having a control valve (17) coupled to the flowline (14; 102) for selectively directing the formation fluid from the flowline (14; 102) into the collection chamber (18) or the wellbore (21).

**9.** Apparatus according to claim 5, 6 or 7, further having a two-way telemetry system for transmitting data between the control circuit (200) and a surface equipment.

**10.** Apparatus according to claim 5, 6, 7 or 9, which is in modular form and comprises:

(a) a probe section (26) having said probe (29);
(b) a pump section (12) having said pump (13) coupled to the flowline (14; 102);
(c) an acoustic density cell (15) coupled to the pump section (12) and the flowline (14; 102); and
(d) a control circuit (200) coupled to the acoustic density cell (15) for processing acoustic signals received therein.

**11.** A system for analysing and collecting formation fluid from a wellbore apparatus as claimed in any of claims 1 to 10, and a surface control unit (50) coupled to the apparatus, said surface control unit (50) having a computer for receiving the processed acoustic signals form the apparatus and determining the fluid density and fluid compressibility therefrom, said control unit (50) transmitting command signals to apparatus for controlling the flow rate into the flowline (14; 102) as a function of the fluid density and compressibility measurement so as to maintain the pressure in the flowline (14; 102) above the bubble point pressure of any gas contained in the formation fluid; and a two-way telemetry system for transmitting data between the downhole formation testing tool (24) and the surface control unit (50).

**12.** A method of retrieving a formation fluid from a zone of interest (22) in a wellbore (21), said method comprising the steps of:

(a) withdrawing formation fluid from the zone of interest (22) into a flowline (14; 102).
(b) determining the speed of sound in the fluid, acoustic impedance and acoustic absorption coefficient of the fluid flowing through the flowline (14; 102);
(c) determining the density and compressibility of the fluid from the speed of sound in the fluid, acoustic impedance and the acoustic absorption coefficient of the fluid flowing through the flowline (14; 102); and
(d) controlling the flow of the fluid into the flowline (14; 102) based on the fluid density and the compressibility of the fluid flowing through the flowline (14; 102) so as to ensure that the pressure in the flowline (14; 102) remains above the bubble point of the fluid.

**13.** A method of determining the bubble point pressure of a formation fluid in a wellbore (21), comprising:

(a) discharging the formation fluid from a zone of interest (22) into a flowline (14; 102) the formation fluid flow rate into the flowline preferably being controlled by a pump by remote means;
(b) determining the speed of sound is the fluid, acoustic impedance and acoustic absorption coefficient of the fluid flowing through the flowline (14; 102)
(c) determining the density and compressibility of the fluid from the speed of sound in the fluid, acoustic impedance and the acoustic absorption coefficient of the fluid flowing through the flowline (14; 102)
(d) changing the fluid flow rate into the flowline (14; 102) and
(e) repeating steps (b)-(d) a desired number of times, recording the pressure in the flowline (14; 102) and the compressability corresponding to each flow rate and determining therefrom the bubble point pressure of the fluid.

**Patentansprüche**

1. Vorrichtung zum Untersuchen von Formationsflüssigkeit aus einer Bohrloch-Verzweigungsstelle, die folgendes umfasst:

   a) eine Sonde (29), die so ausgelegt ist, dass sie an die Formation (22) angelegt werden kann und die Formationsflüssigkeit in eine Feldleitung (14; 102) abzieht, die für das Auffangen von Formationsflüssigkeit verwendet wird,
   b) eine Pumpe (13) für das Steuern der Strömungsgeschwindigkeit der Formationsflüssigkeit in die Feldleitung (14; 102) und
   c) eine akustische Einrichtung (15; 100) für das Bereitstellen von Signalen zum Bestimmen von Eigenschaften der Formationsflüssigkeit in der Feldleitung (14; 102), wobei die akustische Einrichtung (15; 100) einen Signalwandler (108) für das Übertragen akustischer Signale durch die Flüssigkeit in der Feldleitung (14; 102) und einen Empfänger (116) für das Erfassen von durch die Flüssigkeit in der Feldleitung (14; 102) hindurchgehenden akustischen Signalen aufweist.

2. Vorrichtung nach Anspruch 1, bei der die akustische Einrichtung (15; 100) weiterhin eine erste Verzögerungsleitung (104) aufweist, die zwischen dem akustischen Sender (108) und der Feldleitung (14; 102) angeordnet ist, so dass die akustischen Signale durch die Verzögerungsleitung (104) hindurchgehen müssen, bevor sie die Feldleitung (14; 102) erreichen.

3. Vorrichtung nach Anspruch 2, bei der die akustische Einrichtung (100) weiterhin eine zweite Verzögerungsleitung (124) aufweist, die zwischen dem akustischen Empfänger (116) und der Feldleitung (14; 102) angeordnet ist, so dass die akustischen Signale vom akustischen Sender (108) durch die Verzögerungsleitung (124) hindurchgehen müssen, bevor sie den Empfänger (116) erreichen.

4. Vorrichtung nach Anspruch 3, bei der die Verzögerungen in der ersten (104) und der zweiten (124) Verzögerungsleitung in ihrer Beschaffenheit und ihrem Ausmaß im Wesentlichen identisch sind.

5. Vorrichtung nach einem der Ansprüche 1 bis 4, die weiterhin eine Steuerschaltung (200) für das Bestimmen der Eigenschaften der Flüssigkeit in der Feldleitung (14; 102) aufweist, wobei die Steuerschaltung (200) vorzugsweise die Durchflussmenge der Flüssigkeit in die Feldleitung (14; 102) in Abhängigkeit von den bestimmten Eigenschaften der Flüssigkeit steuert, wobei es sich bei den Flüssigkeitseigenschaften vorzugsweise um die Dichte oder die Kompressibilität der Flüssigkeit in der Feldleitung (14; 102) handelt.

6. Vorrichtung nach Anspruch 5, bei der die Steuerschaltung (200) die Durchflussmenge der Formationsflüssigkeit so steuert, dass der Druck in der Feldleitung (14; 102) über dem des Blasenpunktes jedes beliebigen Gases in der Formationsflüssigkeit in der Feldleitung (14; 102) bleibt.

7. Vorrichtung nach Anspruch 6, bei der die Steuerschaltung (200) mit der Pumpe (13) wirkverbunden ist und die Pumpe steuert, um die Strömungsgeschwindigkeit der Formationsflüssigkeit in die Feldleitung (14; 102) in Abhängigkeit von der Flüssigkeitsdichte und - kompressibilität so zu steuern, dass der Feldleitungsdruck über dem des Blasenpunktes jedes beliebigen in der Formationsflüssigkeit in der Feldleitung (14; 102) enthaltenen Gases bleibt.

8. Vorrichtung nach einem der Ansprüche 1 bis 7, die weiterhin eine Kammer (18) für das Aufnehmen von Formationsflüssigkeit aus der Feldleitung (14; 102) sowie vorzugsweise ein Steuerventil (17) aufweist, das mit der Feldleitung (14; 102) verbunden ist, um die Formationsflüssigkeit aus der Feldleitung (14; 102) gezielt in die Sammelkammer (18) oder das Bohrloch (21) zu leiten.

9. Vorrichtung nach Anspruch 5, 6 oder 7, die weiterhin ein 2-Wege-Telemetriesystem für das Übertragen von Daten zwischen der Steuerschaltung (200) und einem Übertage-Gerät aufweist.

10. Vorrichtung nach Anspruch 5, 6, 7 oder 9, die in Modulform vorliegt und folgendes umfasst:

(a) einen Sondenabschnitt (26) mit der Sonde (29),
(b) einen Pumpenabschnitt (12) mit der Pumpe (13), die mit der Feldleitung (14; 102) verbunden ist,
(c) eine mit dem Pumpenabschnitt (12) und der Feldleitung (14; 102) verbundene Zelle (15) für das Messen der Schalldichte und
(d) eine mit der Zelle (15) für das Messen der Schalldichte verbundene Steuerschaltung (200) für das Verarbeiten von darin empfangenen akustischen Signalen.

11. System für das Analysieren und Sammeln von Formationsflüssigkeit aus einer Bohrlochvorrichtung nach einem der Ansprüche 1 bis 10 und eine mit der Vorrichtung verbundene Übertage-Steuereinheit (50), die einen Rechner für das Empfangen der verarbeiteten akustischen Signale aus der Vorrichtung und das Bestimmen der Flüssigkeitsdichte und der Flüssigkeitskompressibilität daraus aufweist und Befehlssignale an die Vorrichtung überträgt, um die Durchflussmenge in die Feldleitung (14; 102) in Abhängigkeit vom Flüssigkeitsdichte- und - kompressibilitätsmesswert so zu steuern, dass der Druck in der Feldleitung (14; 102) über dem des Blasenpunktes jedes beliebigen in der Formationsflüssigkeit enthaltenen Gases bleibt, sowie ein 2-Wege-Telemetriesystem für das Übertragen von Daten zwischen dem Untertage-Formationsuntersuchungswerkzeug (24) und der Übertage-Steuereinheit (50).

12. Verfahren für das Auffangen einer Formationsflüssigkeit aus einem Bereich von Interesse (22) in einem Bohrloch (21), das folgende Schritte umfasst:

(a) Abziehen von Formationsflüssigkeit aus dem Bereich von Interesse (22) in eine Feldleitung (14; 102),
(b) Bestimmen der Schallgeschwindigkeit in der Flüssigkeit, der Schallimpedanz und des Schallabsorptionsgrades der durch die Feldleitung (14; 102) fließenden Flüssigkeit,
(c) Bestimmen der Dichte und Kompressibilität der Flüssigkeit aus der Schallgeschwindigkeit in der Flüssigkeit, der Schallimpedanz und dem Schallabsorptionsgrad der durch die Feldleitung (14; 102) fließenden Flüssigkeit und
(d) Steuern der Durchflussmenge der Flüssigkeit in die Feldleitung (14; 102) auf der Grundlage der Flüssigkeitsdichte und der Kompressibilität der durch die Feldleitung (14; 102) fließenden Flüssigkeit, um sicherzustellen, dass der Druck in der Feldleitung (14; 102) über dem Blasenpunkt der Flüssigkeit bleibt.

13. Verfahren zum Bestimmen des Blasenpunktdrucks einer Formationsflüssigkeit in einem Bohrloch (21), das folgendes umfasst:

(a) Pumpen der Formationsflüssigkeit aus einem Bereich von Interesse (22) in eine Feldleitung (14; 102), wobei die Strömungsgeschwindigkeit der Formationsflüssigkeit in die Feldleitung vorzugsweise über externe Mittel durch eine Pumpe gesteuert wird,
(b) Bestimmen der Schallgeschwindigkeit in der Flüssigkeit, der Schallimpedanz und des Schallabsorptionsgrades der durch die Feldleitung (14; 102) fließenden Flüssigkeit,
(c) Bestimmen der Dichte und Kompressibilität der Flüssigkeit aus der Schallgeschwindigkeit in der Flüssigkeit, der Schallimpedanz und dem Schallabsorptionsgrad der durch die Feldleitung (14; 102) fließenden Flüssigkeit,
(d) Ändern der Strömungsgeschwindigkeit der Flüssigkeit in die Feldleitung (14; 102) und
(e) Wiederholen der Schritte (b)-(d) so oft wie gewünscht, wobei man den Druck in der Feldleitung (14; 102) und die jeder Strömungsgeschwindigkeit entsprechende Kompressibilität aufzeichnet und daraus den Blasenpunktdruck der Flüssigkeit bestimmt.

**Revendications**

1. Appareil pour l'essai de fluides de formation à partir d'une jonction de trous de forage, comprenant :

(a) une sonde (29) adaptée pour être placée contre la formation (22) pour extraire le fluide de formation et l'introduire dans une conduite d'écoulement (14 ;102) utilisée pour récupérer ledit fluide de formation ;
(b) une pompe (13) pour réguler le débit d'arrivée du fluide de formation dans la conduite d'écoulement (14 ; 102) ; et
(c) un dispositif acoustique (15 ; 100) pour fournir des signaux afin de déterminer les caractéristiques du fluide de formation dans la ligne d'écoulement (14 ; 102), ledit dispositif acoustique (15 ; 100) ayant un transducteur

(108) pour transmettre des signaux acoustiques à travers le fluide contenu dans la conduite d'écoulement (14 ; 102) et un récepteur (116) pour détecter les signaux acoustiques qui se propagent à travers le fluide dans la conduite d'écoulement (14 ; 102).

**2.** Appareil selon la revendication 1, dans lequel le dispositif acoustique (15 ; 100) est de plus pourvu d'une première ligne à retard (104) placée entre l'émetteur acoustique (108) et la conduite d'écoulement (14 ; 102) de sorte que les signaux acoustiques passent dans la ligne à retard (104) avant d'atteindre la conduite d'écoulement (14 ; 102).

**3.** Appareil selon la revendication 2, dans lequel le dispositif acoustique (100) est pourvu d'une seconde ligne à retard (124) placée entre le récepteur acoustique (116) et la conduite d'écoulement (14 ; 102) de sorte que les signaux acoustiques qui proviennent de l'émetteur acoustique (108) passent dans la ligne à retard (124) avant d'atteindre le récepteur (116).

**4.** Appareil selon la revendication 3, dans lequel les retards dans les première (104) et seconde (124) lignes à retard ont une composition et une longueur sensiblement identiques.

**5.** Appareil selon l'une quelconque des revendications 1 à 4, étant de plus pourvu d'un circuit de contrôle (200) pour déterminer les caractéristiques du fluide que contient la conduite d'écoulement (14 ; 102), ledit circuit de contrôle (200) régulant préférentiellement l'écoulement d'arrivée du fluide dans la conduite d'écoulement (14 ; 102) en fonction des caractéristiques déterminées du fluide, lesdites caractéristiques du fluide étant préférentiellement la densité du fluide dans la conduite d'écoulement (14 ; 102) ou la compressibilité du fluide dans la conduite d'écoulement (14 ; 102).

**6.** Appareil selon la revendication 5, dans lequel le circuit de contrôle (200) régule l'écoulement du fluide de formation de sorte à maintenir la pression dans la conduite d'écoulement (14 ; 102) au-dessus de la pression au point de bulle de tout gaz éventuellement présent dans le fluide de formation à l'intérieur de la conduite d'écoulement (14 ; 102).

**7.** Appareil selon la revendication 6, dans lequel, pendant le fonctionnement, le circuit de contrôle (200) est couplé à la pompe (13) et contrôle la pompe de sorte à réguler le débit d'arrivée du fluide de formation dans la conduite d'écoulement (14 ; 102) en fonction de la densité du fluide et de sa compressibilité afin de maintenir la pression dans la conduite d'écoulement (14 ; 102) au-dessus de la pression au point de bulle de tout gaz éventuellement présent dans le fluide de formation que contient la conduite d'écoulement (14 ; 102).

**8.** Appareil selon l'une quelconque des revendications 1 à 7, pourvu de plus d'une chambre (18) pour recevoir du fluide de formation à partir de la conduite d'écoulement (14 ; 102), ledit appareil étant en outre préférentiellement équipé d'une soupape de sûreté (17) couplée à la conduite d'écoulement (14 ; 102) pour diriger sélectivement le fluide de formation à partir de la conduite d'écoulement (14 ; 102) jusque dans la chambre collectrice (18) ou le trou de forage (21).

**9.** Appareil selon la revendication 5, 6 ou 7, équipé de plus d'un système bidirectionnel de télémesure pour transmettre des données entre le circuit de contrôle (200) et un équipement en surface.

**10.** Appareil selon la revendication 5, 6, 7 ou 9, qui revêt une forme modulaire et qui comprend :

(a) une section de captage qui comprend ladite sonde (29) ;
(b) une section de pompage (12) dont la pompe (13) est couplée à la conduite d'écoulement (14 ; 102) ;
(c) une cellule de mesure de densité sonore (15) couplée à la section de pompage (12) et à la conduite d'écoulement (14 ; 102) ; et
(d) un circuit de contrôle (200) couplé à la cellule de mesure de densité sonore (15) pour traiter les signaux acoustiques reçus dans cette cellule.

**11.** Système pour l'analyse et la collecte de fluide de formation à partir d'un appareil pour trous de forage selon l'une quelconque des revendications 1 à 10, et une unité de contrôle en surface (50) couplée à l'appareil, ladite unité de contrôle en surface (50) étant équipée d'un ordinateur pour la réception des signaux acoustiques traités provenant de l'appareil et la détermination, à partir de ces signaux, de la densité du fluide et de la compressibilité du fluide, ladite unité de contrôle (50) transmettant à l'appareil des signaux de commande pour réguler le débit d'arrivée dans la conduite d'écoulement (14 ; 102) en fonction des mesures de densité et de compressibilité du fluide,

de sorte à maintenir la pression dans la conduite d'écoulement (14 ; 102) au-dessus de la pression au point de bulle de tout gaz éventuellement contenu dans le fluide de formation ; et un système bidirectionnel de télémesure pour transmettre des données entre l'outil d'essai de formation au fond (24) et l'unité de contrôle en surface (50).

**12.** Procédé pour récupérer un fluide de formation à partir d'une zone d'intérêt (22) dans un trou de forage (21), ledit procédé comportant les étapes consistant à :

(a) extraire du fluide de formation à partir de la zone d'intérêt (22) et l'introduire dans une conduite d'écoulement (14 ; 102) ;
(b) déterminer la vitesse du son dans le fluide, l'impédance acoustique et le coefficient d'absorption acoustique du fluide qui s'écoule dans la conduite d'écoulement (14 ; 102) ;
(c) déterminer la densité et la compressibilité du fluide à partir de la vitesse du son dans le fluide, l'impédance acoustique et le coefficient d'absorption acoustique du fluide qui s'écoule dans la conduite d'écoulement (14 ; 102) ; et
(d) réguler l'écoulement d'arrivée du fluide dans la conduite d'écoulement (14 ; 102) sur la base de la densité du fluide et de la compressibilité du fluide qui s'écoule dans la conduite d'écoulement (14 ; 102) de sorte à garantir que la pression à l'intérieur de la conduite d'écoulement (14 ; 102) demeure supérieure au point de bulle du fluide.

**13.** Procédé pour déterminer la pression au point de bulle d'un fluide de formation dans un trou de forage (21), le procédé consistant à :

(a) décharger du fluide de formation à partir d'une zone d'intérêt (22) et l'introduire dans une conduite d'écoulement (14 ; 102), le débit d'arrivée du fluide dans la conduite d'écoulement étant préférentiellement régulé par une pompe, sous télécommande ;
(b) déterminer la vitesse du son dans le fluide, l'impédance acoustique et le coefficient d'absorption acoustique du fluide qui s'écoule dans la conduite d'écoulement (14 ; 102) ;
(c) déterminer la densité et la compressibilité du fluide à partir de la vitesse du son dans le fluide, de l'impédance acoustique et du coefficient d'absorption acoustique du fluide qui s'écoule dans la conduite d'écoulement (14 ; 102) ;
(d) changer le débit d'arrivée du fluide dans la conduite d'écoulement (14 ; 102) ; et
(e) reprendre les étapes (b) à (d) un nombre souhaité de fois, en enregistrant la pression dans la conduite d'écoulement (14 ; 102) et la compressibilité qui correspond à chaque débit et en déterminant, à partir de celles-ci, la pression au point de bulle du fluide.

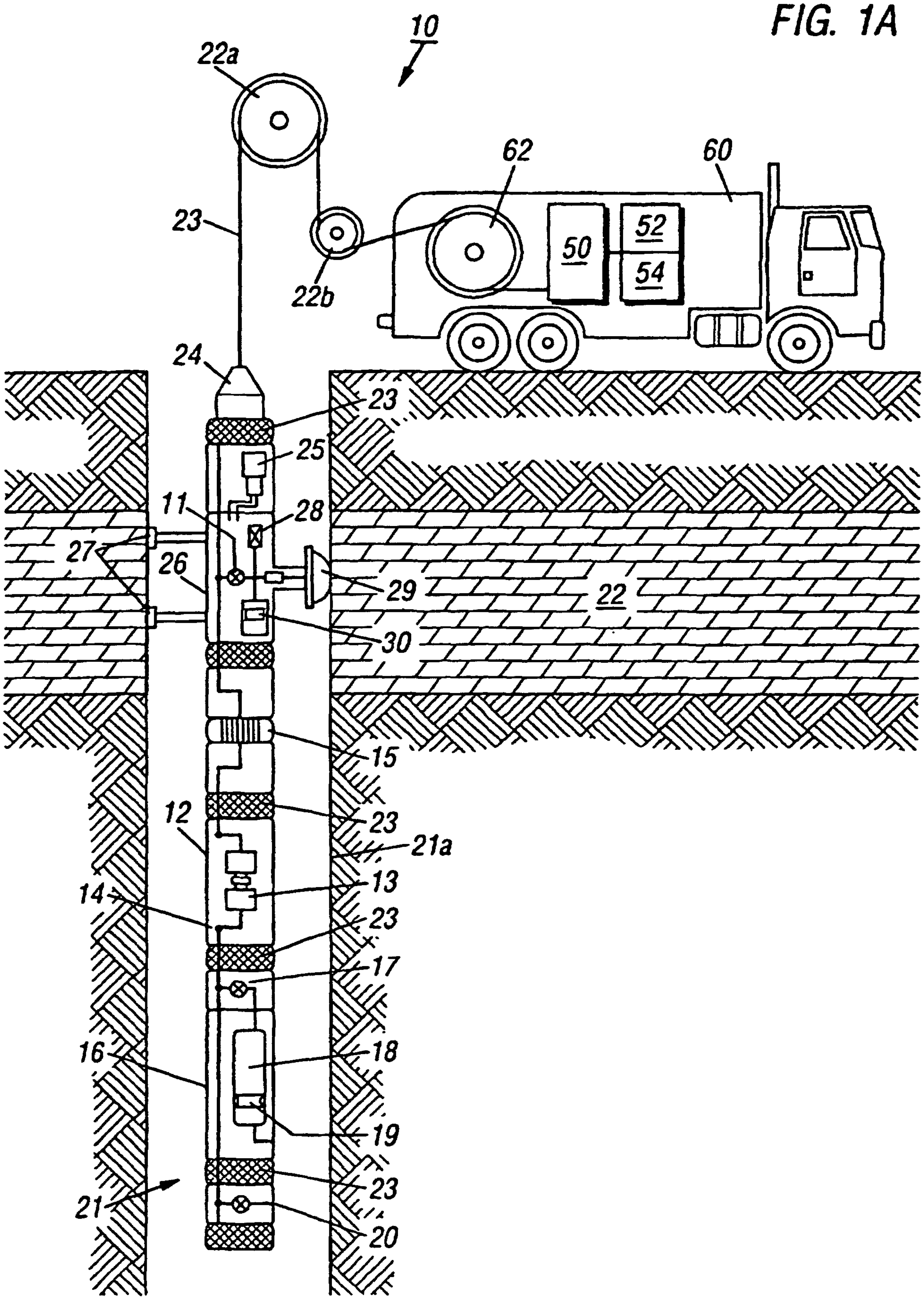
FIG. 1A
10
22a
62
60
23
52
50
54
22b
24
23
25
11
28
27
26
29
22
30
15
23
12
21a
13
14
23
17
16
18
19
23
21
20

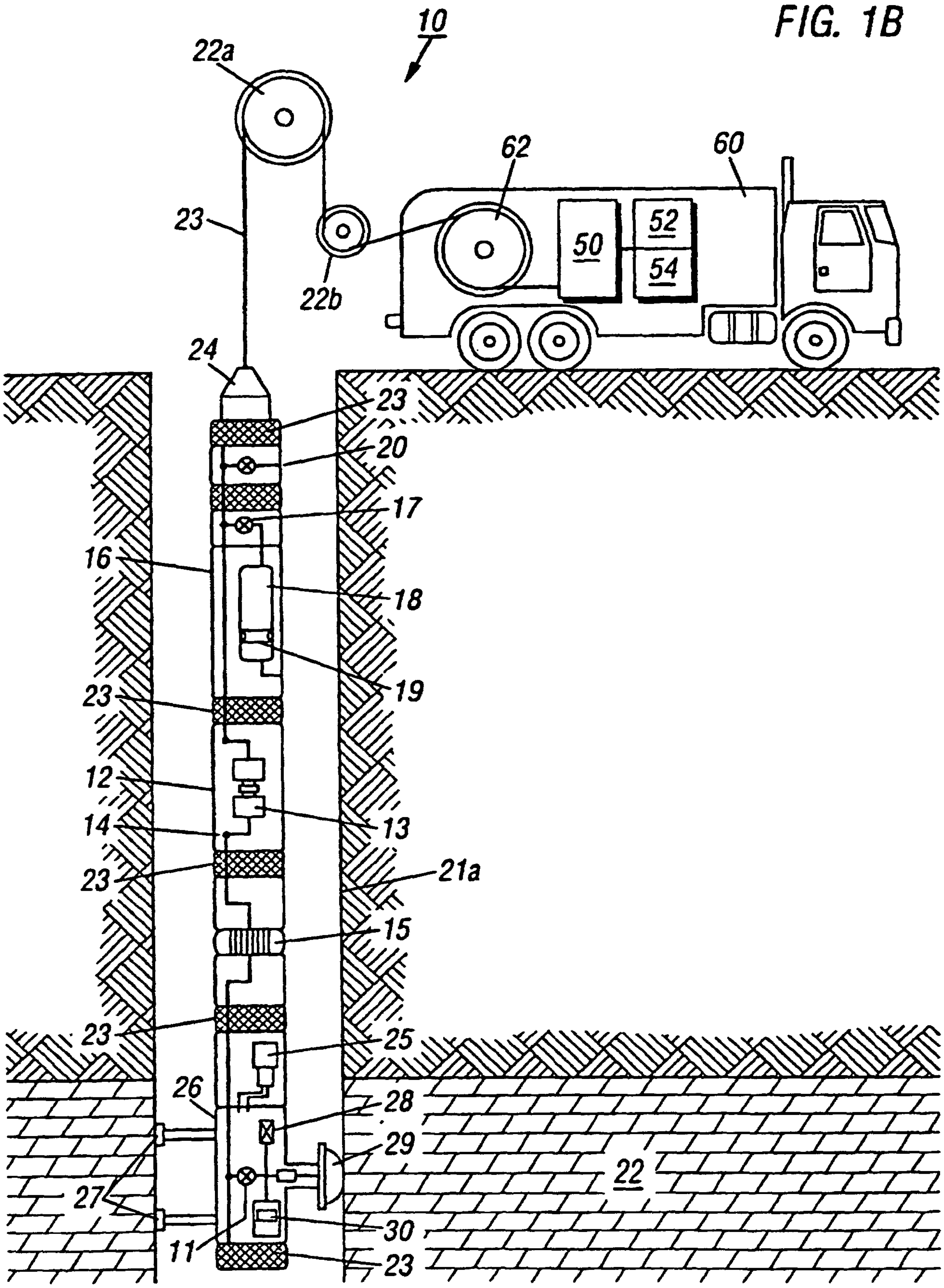

10
22a
23
22b
62
60
50
52
54
24
23
20
17
16
18
19
23
12
14
13
23
21a
15
23
25
26
28
29
22
27
11
30
23


FIG. 1B

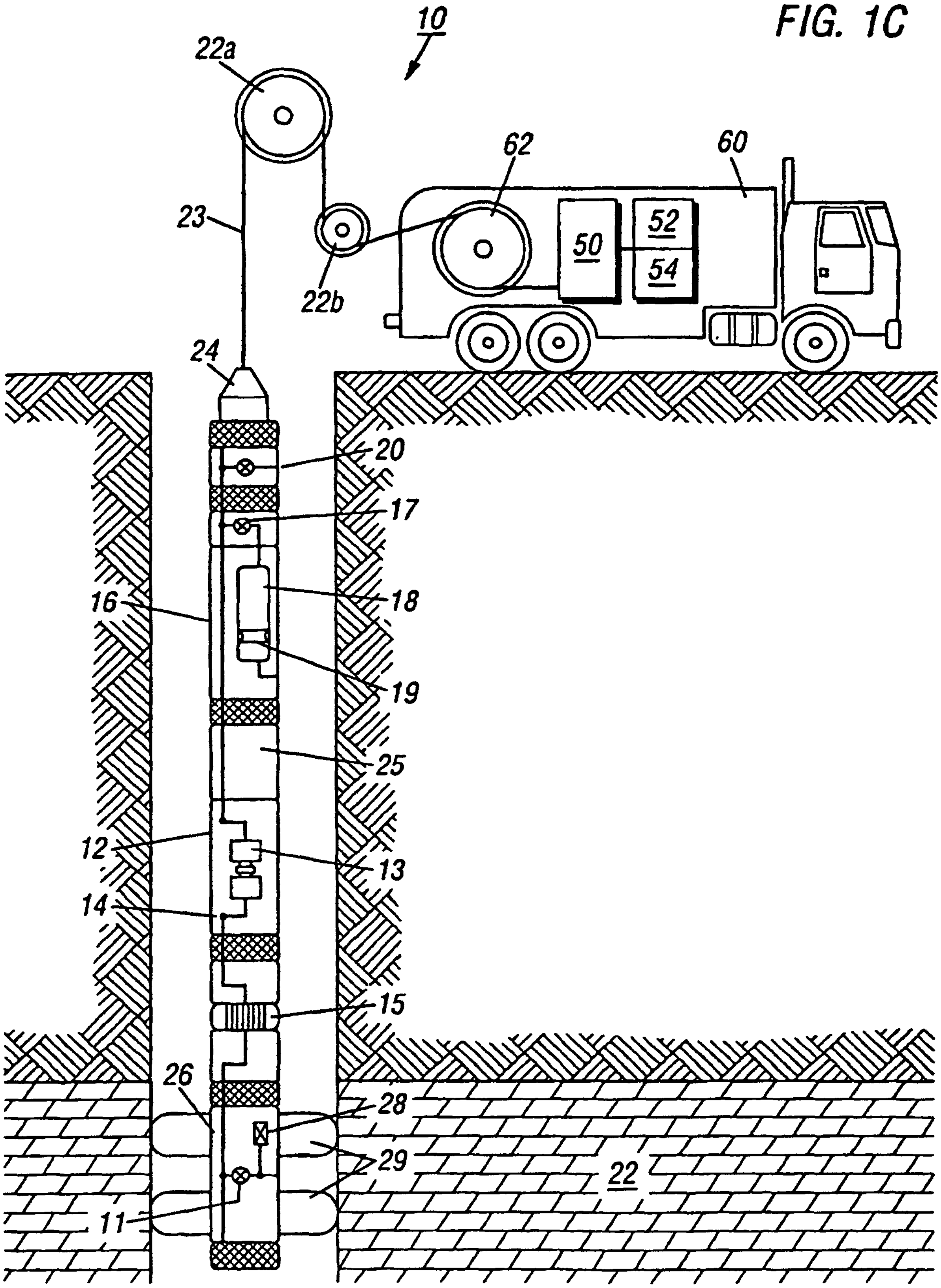
10
22a
23
22b
62
60
50
52
54
24
20
17
16
18
19
25
12
13
14
15
26
28
29
22
11

FIG. 1C

FIG. 2

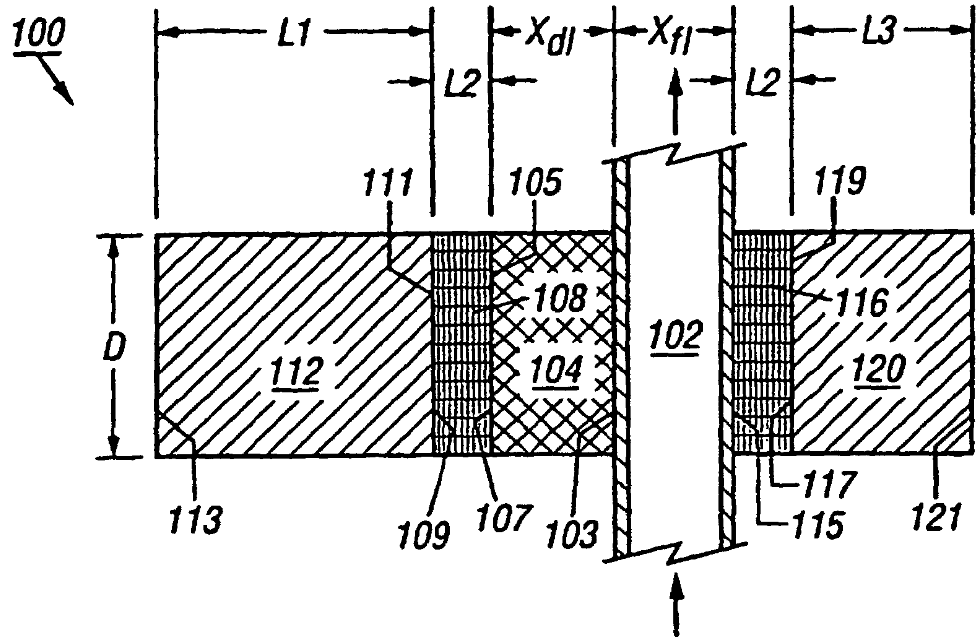
100
L1
L2
$X_{dl}$
$X_{fl}$
L2
L3
111
105
119
108
116
D
112
104
102
120
113
109
107
103
117
115
121

FIG. 3

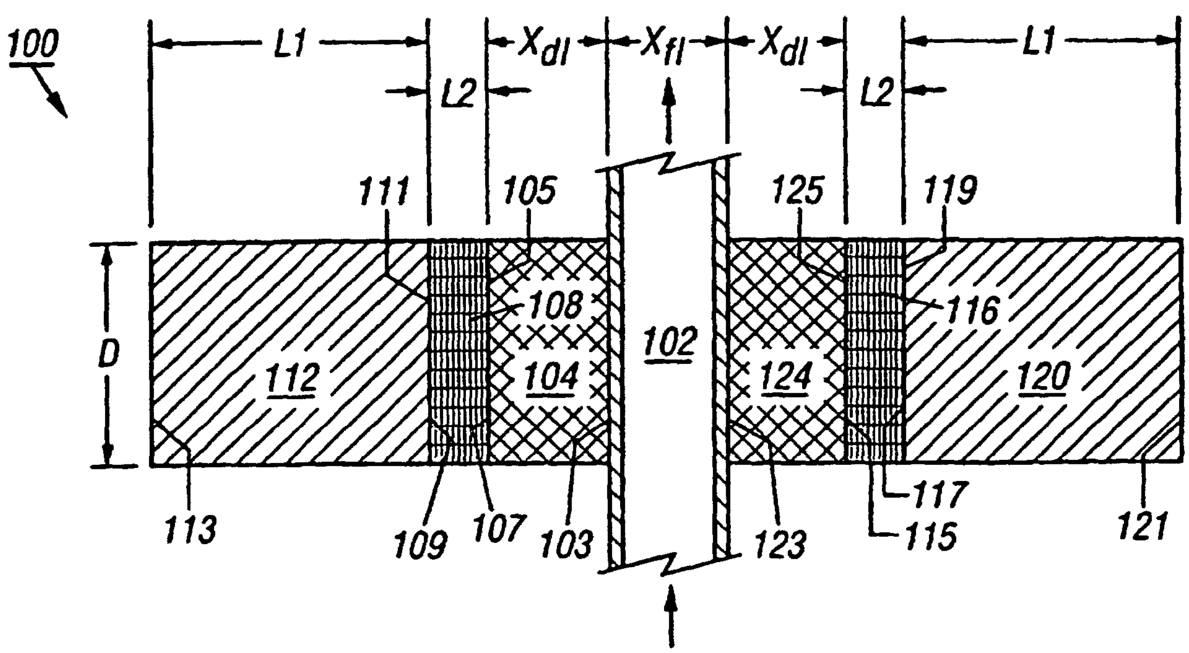
100
L1
L2
$X_{dl}$
$X_{fl}$
$X_{dl}$
L2
L1
111
105
125
119
108
116
D
112
104
102
124
120
113
109
107
103
123
117
115
121

FIG. 4A

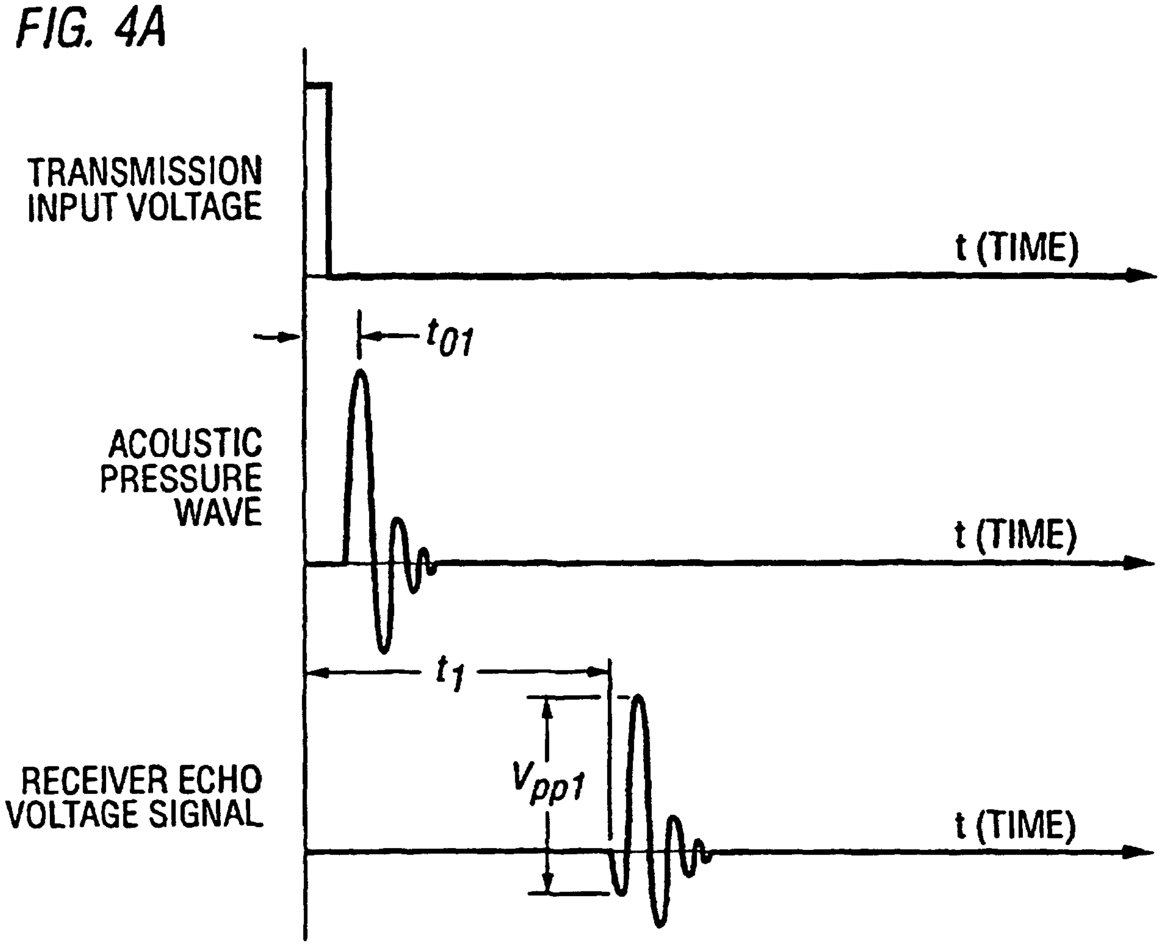
TRANSMISSION
INPUT VOLTAGE
t (TIME)
t01
ACOUSTIC
PRESSURE
WAVE
t (TIME)
t1
Vpp1
RECEIVER ECHO
VOLTAGE SIGNAL
t (TIME)

FIG. 4B

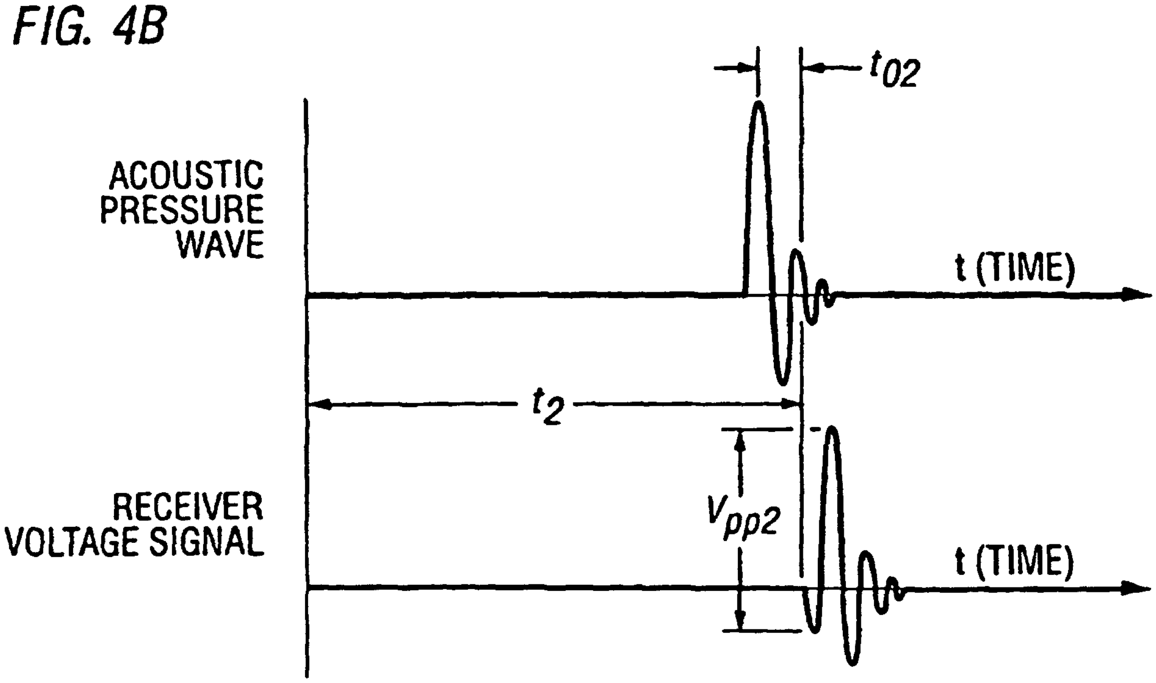
t02
ACOUSTIC
PRESSURE
WAVE
t (TIME)
t2
Vpp2
RECEIVER
VOLTAGE SIGNAL
t (TIME)

FIG. 5

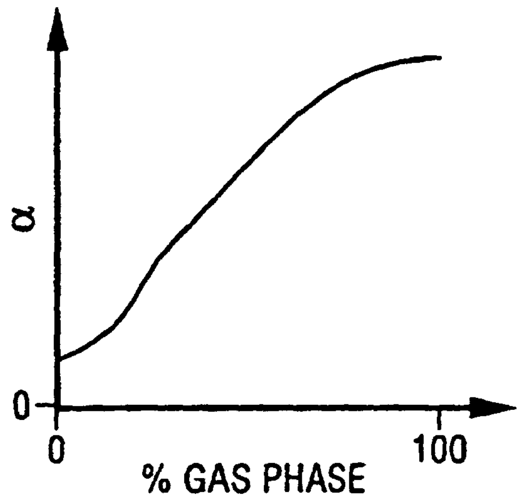
α
0
0
100
% GAS PHASE

FIG. 6

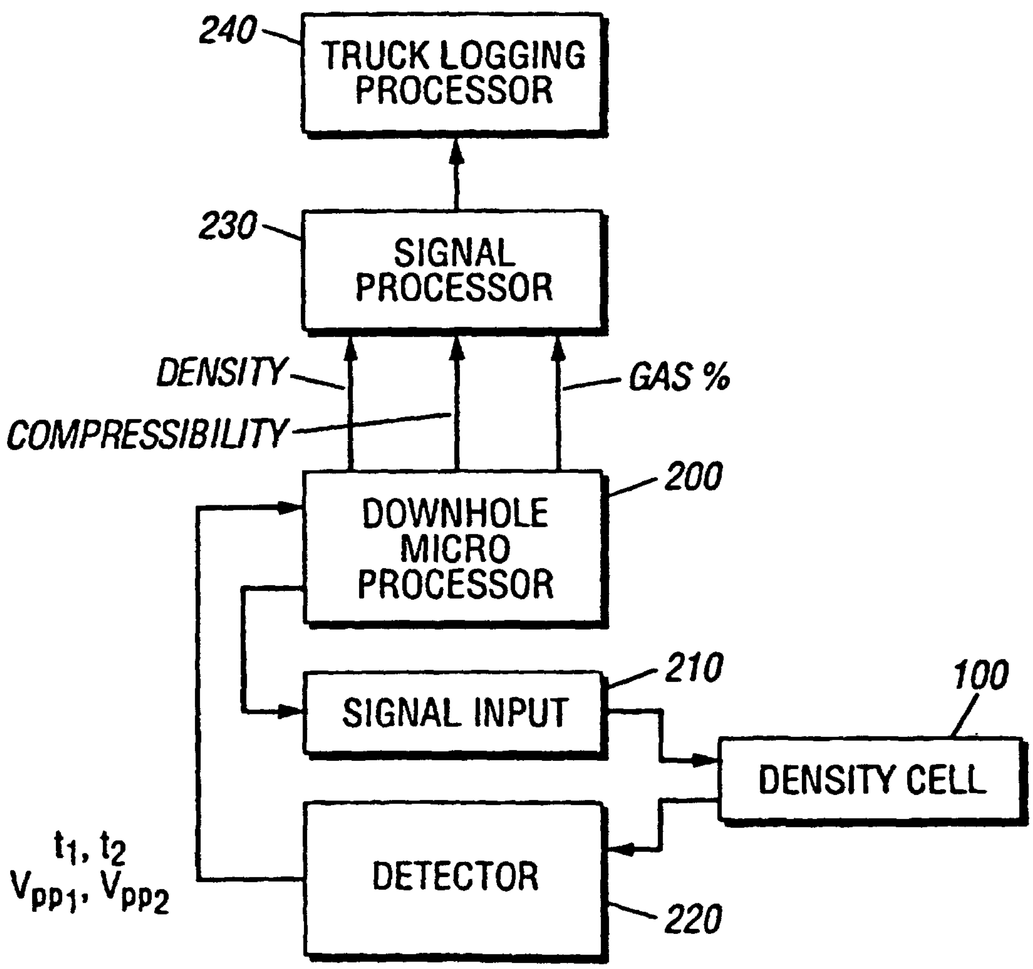
240
TRUCK LOGGING PROCESSOR
230
SIGNAL PROCESSOR
DENSITY
COMPRESSIBILITY
GAS %
200
DOWNHOLE MICRO PROCESSOR
210
SIGNAL INPUT
100
DENSITY CELL
t1, t2
Vpp1, Vpp2
DETECTOR
220